Europäisches Patentamt

(19) European Patent Office

Office européen des brevets

(11) Publication number: **0 007 214**
**B1**

(12) **EUROPEAN PATENT SPECIFICATION**

(45) Date of publication of patent specification: **23.10.85**

(21) Application number: **79301315.2**

(22) Date of filing: **06.07.79**

(51) Int. Cl.⁴: **G 01 N 33/68,** A 61 K 49/00,
G 01 N 33/58

(54) Product for detecting the presence of cancerous or malignant tumor cells.

(30) Priority: **07.07.78 US 922799**
**13.09.78 US 941940**

(43) Date of publication of application:
**23.01.80 Bulletin 80/02**

(45) Publication of the grant of the patent:
**23.10.85 Bulletin 85/43**

(84) Designated Contracting States:
**AT BE CH DE FR GB IT LU NL SE**

(56) References cited:
**DE-A-1 903 050**
**DE-A-1 919 077**
**DE-A-2 546 670**
**DE-A-2 606 257**
**GB-A-1 524 221**
**GB-A-1 532 803**
**US-A-3 798 131**
**US-A-3 960 827**

(73) Proprietor: **Bogoch, Samuel, Dr.**
**46 East 91st Street**
**New York, New York 10028 (US)**

(72) Inventor: **Bogoch, Samuel, Dr.**
**46 East 91st Street**
**New York, New York 10028 (US)**

(74) Representative: **Allard, Susan Joyce et al**
**BOULT, WADE & TENNANT 27 Furnival street**
**London EC4A 1PQ (GB)**

Courier Press, Leamington Spa, England.

**Description**

The present invention relates to a product for detecting the presence of cancerous or malignant tumour cells in a cell collection, and more particularly relates to a product herein termed anti-malignin TAG product (Target Attaching Globulins) in combination with a signal-emitting means. This product may be applied to a cell collections and the anti-malignin TAG product preferentially attaches to cancerous or malignant cells, so detecting them by means of a signal emitting means.

Target attaching globulins are derivatives of compounds herein termed recognins.

As described in DE—A—2,606,257 and DE—A—2,546,670 recognins are made by treating abnormal cells or tissues, for example, tumour cells or artificial cancer cells and separating out the desired products. By the term "recognin" as used herein and throughout the specification is meant an abnormal cell or tissue associated compound which is characterised by:—

(a) being capable of forming a single line precipitate with its specific antibody in quantitative precipitation tests and Ouchterlony gel diffusion test;

(b) being soluble in water and aqueous solutions at acid or neutral pH;

(c) being insoluble in aqueous solutions at alkaline pH;

(d) having a spectrophotometric absorption peak wave length of 280 mμ;

(e) having a molecular weight of from 3,000 to 25,000; and

(f) having an amino acid residue composition in which there is a high proportion of glutamic acid and aspartic acid with respect to the total amino acid residue composition and a high ratio of these acids to histidine.

Again as described in DE—A—2,606,257 and DE—A—2,546,670 recognins may be used to prepare their chemoreciprocals, i.e., by contacting the recognins or the recognins on a support with body fluids. The chemoreciprocals are useful for diagnostic and therapeutic purposes, i.e. for diagnosing and treating cancers. The chemoreciprocals are substances which react with immunochemical-like specificity with a recognin *in vivo* and *in vitro*, i.e. in quantitative precipitation tests, in Ouchterlony double diffusion or in immunofluorescence.

One Recognin is Malignin. Malignin is produced from artificial cancer cells, cancer cells grown *in vitro*. Malignin has a molecular weight of about 10,000 and has a very high percentage of glutamic acid and aspartic acid and high ratios of these acids to histidine. A further analysis of Malignin is provided below.

It is characterized by forming a single line precipitate with its specific antibody in quantitative precipitin tests and Ouchterlony gel diffusion tests, being soluble in water and aqueous solutions having an acid or neutral pH, and insoluble at an alkaline pH, having a spectrophotometric absorption peak wave length of 280 mμ and having a molecular weight of about 10,000.

Recognins are further characterized by being capable of complexing with bromoacetylcellulose to form bromoacetylcellulose-Recognin and producing the specific antibodies anti-Recognin upon injection into mammals, said anti-recognin attaching specifically to the recognin precursor *in situ*.

The anti-Recognins, anti-Malignin is toxic to brain tumor cells *in vitro*. Anti-recognins are antibodies to the group of antigens which are herein termed recognins, and they form a single line precipitate with Recognins in quantitative precipitation tests and Ouchterlony gel diffusion test.

Recognins, such as Malignin have various uses as discussed in DE—A—2,606,257 and DE—A—2,546,670.

As disclosed in DE—A—2,606,257 and DE—A—2,546,670, Recognins may also be used to produce Target reagents which comprise the complexes of the Recognin with a carrier to facilitate its applicability in biological systems. Thus, for example, the complex conveys the physical-chemical characteristics of the Recognin itself.

As further discussed in the aforementioned German Patent applications a class of Chemoreciprocals term anti-Recognins, e.g. anti-Malignin may be made by injecting the Recognins into a biological system.

An important finding was that another class of chemoreciprocals could be formed by complexing Target reagents with their chemoreciprocals. In accordance with the invention, this type of compound and the anti-malignin may be complexed with and used for the delivery of diagnostic agents to specific cells or sites in a biological system. These compounds may also be used for purification procedures. Target reagents are also useful for increasing the amount of TAG products (described below) in a biological system, such as by bringing an immunologically effective dose of Target into contact with bodily tissues or fluids.

Additional chemoreciprocals are TAG reagents (i.e. Target-Attaching Globulins) and important features of this invention are TAG products produced by bringing Target reagents into contact with body fluids for varying periods of time to form a complex and cleaving TAG therefrom. Two useful embodiments are S-TAG and F-TAG.

A process for producing S-TAG (Slow-Target-Attaching-Globulin) comprises reacting blood serum or other body fluid with Target (e.g. Bromoacetylcellulose-Malignin) for approximately two hours or more at a low temperature, e.g. about 4°C, and cleaving S-TAG from the resulting material, e.g. with dilute acid for approximately two hours at a temperature of about 37°C. The product S-TAG prepared in accordance with this process is characterized by being soluble in aqueous buffered solutions, forming a single line precipitate with its corresponding recognin in Ouchterlony gel diffusion tests, being non-dialyzable in cellophane membrances, being retained by millipore filters which retain molecules over 25,000 molecular weight having molecular weights in different states of aggregation as determined by thin layer gel chromatography of approximately 50,000, and multiples thereof into the macroglobulin range and having a spectrophotometer absorption peak wave length of 280 mμ.

A process for producing F-TAG (Fast-Target-Attaching-Globulin) comprises reacting blood serum or other body fluid with Target (e.g. Bromoacetylcellulose-Malignin) for approximately 10 minutes at a low temperature, e.g. about 4°C, and cleaving F-TAG from the resulting material, e.g. with dilute acid for approximately two hours at a temperature of about 37°C. The product F-TAG prepared in accordance with this process is characterized by being soluble in aqueous buffered solutions, forming a single line precipitate with its corresponding Recognin in Ouchterlony gel diffusion tests, being non-dialyzable in cellophane membranes, being retained by millipore filters which retain molecules over 25,000 molecular weight, having molecular weights in different states of aggregation as determined by thin layer gel chromatography of approximately 50,000, and multiples thereof into the macroglobulin range and having a spectrophotometric absorption peak wave length of 280 mμ. TAG products are useful in this invention for detecting or diagnosing the presence of tumor cells in a histology section and other cell collections, which comprises applying TAG conjugated with a labeling substance namely 99[m] Technetium, to said section, whereby radio-active labeling occurs only with tumor cells. One advantage of the methods disclosed herein is that the utilisation of 99[m] Technetium as the radiolabel attached to the TAG product ensures a high radio-labelling activity and therefore a very sensitive diagnostic method. TAG products additionally have been found to be cytotoxic to tumor cells. TAG products are also useful for directing the delivery of a diagnostic, agent to specific cells or sites by introducing said agent in complexed form with the TAG product.

It has now been discovered that the combination of the TAG product and the 99[m] Technetium radioisotope gives a particularly useful product for effecting the diagnosis of cancer.

Accordingly the present invention provides a product for detecting the presence of cancerous or malignant tumor cells in a cell collection which comprises an anti-malignin TAG product (Target-Attaching-Globulins) in combination with a signal-emitting means which may be applied to said cell collection, whereby said anti-malignin TAG product preferentially attaches to cancerous or malignant cells, and detects said cancerous or malignant tumor cells by means of said signal-emitting means, characterized in that said signal-emitting means is 99[m] Technetium.

The product of the present invention is applicable to both *in vivo* and *in vitro* cell collections and also for detecting both glial and non-glial tumor cells. Advantageously, the TAG product of the present invention is produced by the reaction of anti-malignin serum with a malignin-based reagent, preferably bromoacetylcellulose-malignin.

Normal cell division in plants or animals is restricted or inhibited when the cells come to occupy fully a particular space. The mechanisms (a) by which normal cells "recognize" that they have filled the space available to them, and (b) by which the operation of this recognition mechanism in turn inhibits cell division, have both been unknown. The existence of a group of compounds termed recognins whose precursors are increased in concentration when normal recognition and learning occur, and which relate to recognition and learning particles in cells, and with the connection of cells to each other is known. By attempting to produce these compounds from cancer cells, it has been discovered that changes in their molecular structure occurred at the same time as the cells lost their ability (a) to recognize that they had filled their normal volume, and/or (b) to stop dividing when they had filled their normal volume.

For use in this invention are products termed Target-Attaching-Globulins (TAG). They are produced by two reactions; the first comprising reacting biological fluids with a synthetic complex containing physicochemical configurations which mimic those of the Recognins and which are called TARGET, and the second comprises cleaving the specific TAG from the complex, and by the measure of the TAG so produced obtaining a quantitative indication from the biological fluids of living organisms whether there is present a tumor in that organism; hence a diagnostic test for tumours. Because TAG products and anti-Recognins are physicochemically complimentary to Recognins, they are termed Chemoreciprocals.

It has further been discovered that two quantitatively and qualitatively distinct TAG products can be produced depending upon the time permitted for the reaction of serum with the specific Target reagent used, and depending upon the time permitted for cleavage of the product which has been complexed.

After examining the amounts of these products which could be produced from a number of different individuals with brain tumors and various other medical disorders, as well as in those with no apparent disease process, it became apparent that the amounts of these two new products which could be produced in a given individual was indicative of whether that individual had a brain tumor, hence a serum diagnostic test for brain tumours.

The products of this invention are provided by anti-recognin TAG products in combination with signal emitting means and their utility may be demonstrated by the preferential localisation of the signal in the vicinity of glial and non-glial tumour cells in cell collections e.g. histological sections of, for example, brain tumor and surrounding tissue removed at surgery of the brain tumor. Thus a new product is available for determining through cell collection examination with a new degree of certainty whether tumor cells are present in the tissue removed, and whether these tumor cells have penetrated to the very edge of the tissue removed, indicating the likelihood that tumor still remains in the brain or other organ, or that tumor cells are absent from the periphery of the tissue removed, indicating the possibility that all of the tumor has been removed from the brain or other organ. In addition, TAG and Anti-recognins produced as described have been found to be cytotoxic for glioma brain tumor cells grown in tissue culture *in vitro.* This high affinity culture, is further evidence of the specific-coupling potential of the new product TAG. Coupling of the radioactive tracer, 99$^m$ Technetium, to TAG provides the new TAG product which is useful for example *in vivo* in the diagnosis of tumors and in their exact localization. Thus for example the injection of TAG labelled with 99$^m$ Technetium, either intraarterially or intravenously, into the cerebrospinal fluid, or directly into brain tissue or its cavities, permits the demonstration by radioactive means, of the presence of a brain tumor, for it is only to the tumor cells that the TAG specifically attaches. Further, this product permits the precise visualization of the location of the brain tumor and or other types of tumor. This can be seen to be an improvement of this *in vivo* diagnostic method using anti-Astrocytin produced in rabbit blood to label the brain tumor, because the use of TAG produced from human serum avoids the possibility of foreign protein reactions. This selectivity is universally recognized as the crucial, or at least one crucial factor for achieving effective chemical or physical diagnosis of tumors. Thus the radiolabeled TAG has demonstrated efficiency in attaching preferentially to the tumor cells, and has promise as a new diagnostic product for these reasons.

In the serum of patients with malignant tumours, as will be seen in the examples below, one type of TAG, SLOW-TAG (S-TAG) as distinguished from Fast-Tag (F-TAG) can be produced in relatively greater amounts from a given volume of serum than in patients without such tumors. This suggests that either one of TAG's naturally occurring precursors (P-TAG) is increased in concentration or that other factors exist which favour the relative *in vitro* production of S-TAG over F-TAG.

The possible relationship of the function of the actual synthetic products TARGET and TAG to their precursors, and in turn to functions of postulated but not demonstrated cell "antigens" and circulating "antibodies" to them which may exist *in vivo* has yet to be elucidated. Thus, for example, in antibody-like fashion, F-TAG and S-TAG produce single discrete lines of reaction with Astrocytin in Ouchterlony gel diffusion, and the injection of TARGET in rabbits induces an increase in the yield of TAG products from rabbit serum after reacting with Target. The finding that there may be a normal level of a precursor resembling circulating antibody to a cell antigen which is hidden in the non-dividing cell raises a question as to the possible function of the pair. It is here proposed that TAG precursor (P-TAG) and TARGET-like substances exist *in vivo* which function in the control of cell proliferation and cell death. Thus, for example, the exposure of a cell constituent which normally is not directly exposed to serum proteins may occur during cell division. The exposure of this cell constituent could result in that constituent becoming converted to a TARGET-like substance to which the attachment of a P-TAG-like molecules from serum may then occur, which would stimulate cell division or inhibit it. Alternatively, a non-dividing cell which is injured or malfunctioning may expose a TARGET-like substance to which the attachment of P-TAG-like molecules may be reparative. However, under certain cell conditions the attachment of P-TAG-like molecules may induce the destruction of the cell (e.g. Anti-Glioma-TAG synthetically produced as here described is markedly cytotoxic to glioma tumor cells growing in tussue culture). This could thus represent a mirror of a normal mechanismn for the control of cell division, and for either the repair or the removal of individual cells in the body throughout the life of the organism. If the exposure of cell constituents is abnormally increased so that abnormally large amounts of cell TARGET-like substances are formed, as may occur in rapidly dividing cancer cells such as in brain gliomas, an increase in the concentration of one type of serum P-TAG relative to another may be induced.

Whatever the actual function of the precursors, the increase in the relative amount of predominantly one type of TAG, SLOW-TAG (S-TAG) which can be produced *in vitro* by the methods here described from the serum of patients with malignant tumors is the basis of the serum diagnostic test described in the Examples which follow.

The following Examples 1 to 3 are included to provide relevant procedures for an understanding of the invention herein disclosed. Example 4 is illustrative of the present invention.

The procedures employed to isolate the recognin, Malignin, in a purified form are described in the art, for example in DE—2,546,670. Relatively pure recognins are essential as a starting point from which to manufacture the chemoreciprocals, anti-recognins.

Example 1 gives information concerning the production of TARGET reagent from Malignin which is an essential constituent in the manufacture of TARGET products for use in this invention.

Example 2 discloses the production of the anti-recognins, i.e. the antisera to Malignin and TARGET.

Example 3 illustrates the *in vitro* production of Target-Attaching-Globulins (TAG) from Biological fluids.

Example 4 is illustrative of the invention and discloses the *in vivo* detection of malignant tissue using the radio-isotope 99$^m$ Technetium. The specificity of the TAG products and the efficacy of radiolabelling using this isotope are highly relevant to the practical diagnostic potential demonstrated herein.

Example 1
Production of TARGET reagents from MALIGNIN. MALIGNIN is complexed with a carrier to produce TARGET reagent.

In the preferred embodiment, MALIGNIN is dissolved in 0.15 M $NaH_2PO_4$ — citrate buffer pH 4.0. A

**0 007 214**

bromoacetyl-resin, for example bromoacetylcellulose (BAC) having 1.0 to 1.5 milliequivalents Br per gram of cellulose, stored in the cold, is prepared in 0.15 M $NaH_2PO_4$ buffer, pH 7.2. Convert the buffer to pH 4 by pouring off the pH 7.2 buffer solution and adding 0.15 M $NaH_2PO_4$ — citrate buffer, pH 4.0. The MALIGNIN solution and the BAC solution are stirred together (10:1 BAC to MALIGNIN ratio) for 30 hours at room temperature, then centrifuged.

It is preferred that all sites on the BAC which are available to bind to MALIGNIN be bound. This may be accomplished as follows. The supernatant from the immediately preceding step is lypholized and the protein content determined to indicate the amount of MALIGNIN not yet complexed to BAC. The complexed BAC-MALIGNIN is resuspended in 0.1 M bicarbonate buffer pH 8.9, stirred 24 hours at 4° to permit the formation of chemical bonds between the BAC and the MALIGNIN. After the 24 hours, the suspension is centrifuged and supernatant analyzed for protein. The complexed BAC-MALIGNIN is now resuspended in 0.05 M aminoethanol — 0.1 M bicarbonate buffer pH 8.9 in order to block any unreacted bromine. The suspension is centrifuged and the supernatant is kept but not analysed because of the presence of aminoethanol. Removal of all unbound MALIGNIN is then accomplished by centrifugation and resuspension for three washings in 0.15 M NaCl until no absorbance is measured on the spectrophotometer at 266 mµ. The BAC-MALIGNIN complex is now stirred in 8 M urea for 2 hours at 38°C, centrifuged, then washed (three times usually suffices) with 8 M urea until no absorbance is shown in the washings at 266 mµ. The complex is then washed with 0.15 M NaCl two times to rid of urea. The complex is then stirred at 37°C in 0.25 M acetic acid for 2 hours to demonstrate its stability. Centrifuge and read supernatant at 266 mµ — no absorbance should be present. This chemically complexed BAC-MALIGNIN is therefore stable and can now be used as a reagent in the methods described below; in this stable reagent form it is referred to as TARGET (TOPOGRAPHIC-ANTIGEN-LIKE-REAGENT-TEMPLATE) because it is a synthetically produced complex whose physical and chemical properties mimic the stable cell-bound precursor of MALIGNIN when it is in a potential relative state with serum components. For storing, the TARGET reagent is centrifuged and washed until neutralised with 0.15 M $NaH_2PO_4$ buffer pH 7.2.

TARGET reagents may be prepared from bromoacetyl liganded carriers other than cellulose, such as bromoacetylated resins or even filter paper.


Example 2
Production of antisera to Malignin and TARGET

Antiserum to Malignin or TARGET reagents may be produced by inducing an antibody response in a mammal to them. The following procedure has been found to be satisfactory.

One mg of Malignin is injected into the toe pads of white male rabbits with standard Freud's adjuvant, and then the same injection is made intraperitoneally one week later, again intraperitoneally ten days and, if necessary, three weeks later. Specific antibodies may be detected in the blood serum of these rabbits as early as one week to ten days after the first injection. The same procedure is followed for TARGET antigen by injecting that amount of TARGET which contains 1 mg of Malignin as determined by Folin-Lowry determination of protein.

The specific antibody to Malignin is named Anti-Malignin. Similarly, the specific antibody to TARGET reagent is named Anti-Target.

These antibodies show clearly on standard Ouchterlony gel diffusion test for antigen-antibody reactions with specific single sharp reaction lines produced with their specific antigen.

The presence of specific antibodies in serum can also be tested by the standard quantitative precipitin test for antigen antibody reactions. Good quantitative precipitin curves are obtained and the micrograms specific antibody can be calculated therefrom.

Further evidence of the presence of specific antibodies in serum can be obtained by absorption of the specific antibody Anti-Malignin onto Bromacetyl-cellulose-Malignin (BAC-Malignin) prepared above. The antiserum containing specific Anti-Malignin can be reacted with BAC-Malignin. When the serum is passed over BAC-Malignin only the specific antibodies to Malignin bind to their specific antigen Malignin. Since Malignin is covalently bound to Bromacetyl-cellulose the specific antibody, Anti-Malignin, is now bound to BAC-Malignin to produce BAC-Malignin-Anti-Malignin (BACM-Anti-Malignin). This is proved by testing the remainder of the serum which is washed free from BAC-Malignin. On standard Ouchterlony diffusion no antibodies now remain in the serum which will react with Malignin. It is therefore concluded that all specific antibodies (Anti-Malignin) previously shown to be released from its binding to BAC-Malignin it is thereby isolated free of all contaminating antibodies. This release of Anti-Malignin may be accomplished by washing the BAC-Malignin-Anti-Malignin complex with 0.25 M acetic acid (4°C, 2 hours) which has been shown above not to break the BAC-Malignin bond.

The antibodies to TARGET show clearly on standard Ouchterlony gel diffusion test for antigen-antibody reactions with specific single reaction lines produced with TARGET which show a line of identity with the line of reaction to ANTI-MALIGNIN antisera (i.e. that produced to the injection of MALIGNIN itself). Some rabbits, it has been noted, have levels of ANTI-TARGET in their blood prior to being injected with TARGET. These ANTI-TARGET substances, when reacted specifically with TARGET reagents as to be described in tests of human sera, lead to the production of approximately equivalent amounts of the two types of TAG, S-TAG and F-TAG (see later Examples).

Example 3

Detection of malignant tumors by quantitative production *in vitro* of TARGET-ATTACHING-GLOBULINS (TAG) from biological fluids.

TARGET reagent prepared in accordance with Example 2 is washed to remove any unbound MALIGNIN which may be present due to deterioration. The following procedure is satisfactory. TARGET reagent is stirred for two hours at 37° with acetic acid, centrifuged, the supernatant decanted, and the optical density of the supernatant read at 266 mμ. If there is any absorbance, this wash is repeated until no further material is solubilized. The TARGET is then resuspended in phosphate buffered saline, pH 7.2 (Standard S-TAG and F-TAG purified from previous reactions of human serum by the procedure described below can be used if available, as reference standards to test the TARGET reagent, as can whole rabbit serum which has been determined to contain S-TAG and F-TAG by other preparations).

The Slow-Binding (S-TAG) determination is performed as follows: Frozen serum stored more than a few days should not be used. Serum is carefully prepared from freshly obtained whole blood or other body fluid by standard procedures in the art. The following procedure has been found to be satisfactory. Blood is allowed to clot by standing for 2 hours at room temperature in a glass test tube. The clots are separated from the walls with a glass stirring rod and the blood allowed to stand at 4°C for a minimum of 2 hours (or overnight). The clots are separated from the serum by centrifuging at 20,000 rpm at 4°C for 45 minutes. The serum is decanted into a centrifuge tube and centrifuged again at 2000 rpm at 4°C for 45 minutes. The serum is decanted and a 1% Solution of Methiolate (1 g in 95 ml water and 5 ml 0.2 M bicarbonate buffer pH 10) is added to the extent of 1% of the volume of serum.

Serum samples, prepared by the above or other procedures, of 0.2 ml each are added to each of 0.25 ml aliquots of TARGET suspension reagent containing 100—200 micrograms of MALIGNIN per 0.25 ml TARGET reagent, in duplicate determination. The suspension is mixed at 4°C in a manner to avoid pellet formation. For example, a small rubber cap rapid shaken may be used for 1—2 seconds and then, with the tubes slightly slanted, they may be shaken in a Thomas shaker for about 2 hours or more. The TARGET reagent and protein bound to it are separated from the serum. One of the procedures which has been found to be satisfactory is the following. The tubes are then centrifuged at 2000 rpm for 20 minutes at 4°C, the supernatant decanted, the pellet which is formed by centrifugation washed 3 times by remixing and shaking at room temperature with 0.2—0.3 ml of 0.15 M Saline, centrifuged and the supernatants discarded.

The protein which remains attached to the TARGET is cleaved therefrom and quantitatively determined. For example, 0.2 ml of 0.25 M acetic acid is added, the suspension shaken for 1 to 2 seconds with a rubber cup shaker, then in a Thomas shaker for about 2 hours in a 37°C incubator. The tubes are centrifuged at 2000 rpm for 4°C for 30 minutes. The supernatant is carefully decanted to avoid transferring particles and the optical density of the supernatant is read at 280 mμ. The value of the optical density is divided by a factor of 1.46 for results in micrograms per ml serum protein (S-TAG). Duplicate determinations should not vary more than 5%. Any other procedure effective for determining protein content may be used, such as Folin Lowry determination, but standards must be specified to determine the range of control and tumor values of S-TAG mins F-TAG concentration.

The Fast-Binding (F-TAG) determination is performed as follows: Frozen serum stored more than a few days should not be used. Serum is carefully prepared from freshly obtained whole blood or other body fluid by standard procedures in the art. The procedure given above in this Example for serum preparation is satisfactory.

Serum samples, prepared by the above or other procedures are allowed to stand at 4°C for 10 minutes less than the total time the S-TAG serum determinations were allowed to be in contact with TARGET reagent above [e.g. 1 hour 50 minutes if a "two hour" S-TAG determination was made]. This procedure equilibrates the temperature histories of S-TAG and F-TAG determinations.

Add 0.2 ml samples of the temperature equilibrated serum to each of 0.25 ml aliquots of TARGET suspension reagent containing 100—200 micrograms of MALIGNIN per 0.25 ml TARGET reagent, in duplicate determination. The suspension is then mixed at 4°C for approximately 10 minutes in a manner to avoid pellet formation. For example, a small rubber cap rapid shaken may be used for 1—2 seconds and then, with the tubes slightly slanted, they may be shaken in a Thomas shaker for approximately 10 minutes. The TARGET reagent and protein bound to it are separated from the serum. One of the procedures which has been found to be satisfactory is the following. The tubes are then centrifuged at 2000 rpm for 20 minutes at 4°C, the supernatant decanted, the pellet which is formed by centrifugation washed 3 times by remixing and shaking at room temperature with 0.2—3.0 ml of 0.15 M Saline, centrifuged and the supernatants discarded.

The protein which remains attached to the TARGET is cleaved therefrom and quantitatively determined. The procedure described above in this Example for determining S-TAG concentration is satisfactory. Any other procedure effective for determining protein content may be used, such as Folin-Lowry determination, but standards must be specified to determine the range of control and tumor values of S-TAG minus F-TAG concentration.

The final results are expressed as TAG micrograms per ml of Serum, and equal S-TAG minus F-TAG. TAG values in non-brain-tumor patients and other controls currently range from zero (or a negative number) to 140 micrograms per ml of serum. TAG values in brain tumor patients currently range from 141 to 500 micrograms per ml of serum.

Example 4
Detection of cancer cells with radioisotope signal from TAG.

In this Example, the feasibility of attaching a radioactive label to TAG is demonstrated. Second, the injection into animals of this radio-labeled TAG has been accomplished and shown to be safe and effective. Third, the radio-labeled TAG localized preferentially in the cancer tissue when compared to normal tissue, thus indicating that the specificity previously demonstrated *in vitro* of the preference for cancer cells which is conveyed by the use of specific anti-Malignin TAG products is confirmed *in vivo*.

The labeling of TAG with 99 m technetium ($^{99m}$Tc)
Procedure for labeling
1. Two preparations of TAG were used, here designated TAG-1 and TAG-2. TAG-1 and TAG-2 (concentration of each 0.4 mg/0.5 ml) were added to separate sterile evacuated vials.
2. To each vial was added 0.1 ml of a stannous chloride solution (10 mg $SnCl_2$. 2 $H_2O$ in 100 ml of 0.01 N HCl). The vials were mixed for 3—4 minutes.
3. 0.1 ml (6mCi) of $^{99m}$Te-pertechnetate (sodium salt) was added and mixed 2—3 minutes.

Procedure for determining labeling efficiency
Samples of the $^{99m}$Tc-TAG-1 and $^{99m}$Tc-TAG-2 were tested for labeling efficiency by descending paper chromatography using Watman No. 1 paper with 85% methanol as the solvent. A similar study was done with Sodium Pertechnetate-$^{99m}$TC which acted as a control.
After 2 hours, the papers were removed from the chromatography tank and divided in two sections: (1) 1 cm about the origin; (2) the remaining paer up to the solvent front. Each section was then counted in a gamma well scintillation counter and its content of radioactivity determined (cpm).
Approximately 50 lambda were plated on each paper strip.

Procedure for antigen-antibody reaction
A portion of the labeled solution was also plated on an Ouchterlony gel plate to determine its ability to react with malignin in the antigen-antibody reaction. After a 3 hour period, the resulting sharp reactive lines were removed from the gel and their content of radioactivity measured. An equal portion of the gel not involved in the reaction was also removed and its content of radio activity was also measured as background.

Results
Labeling efficiency

TABLE 1
Labeling efficiency of $^{99m}$Tc-TAG-1 and $^{99m}$Tc-TAG-2

| Compound | Site on paper | CPM | % | Chemical species |
|---|---|---|---|---|
| $NaTcO_4$-99mTc | Origin | $4.94 \times 10^5$ | 7.33% | reduced $TcO_4$- |
| $NaTcO_4$-88mTc | Solvent front | $6.25 \times 10^6$ | 92.67% | $TcO^4$- |
| TAG-1 | Origin | $4.35 \times 10^6$ | 98.47% | TAG-99mTc |
| TAG-1 | Solvent front | $6.76 \times 10^4$ | 1.53% | $TcO_4$- |
| TAG-2 | Origin | $1.96 \times 10^6$ | 98.01% | TAG-99mTc |
| TAG-2 | Solvent front | $3.98 \times 10^4$ | 1.99% | $TcO_4$- |

TABLE 2
Antigen-antibody resection

| Gel area | Counts per min | % |
|---|---|---|
| TAG-2 Line | $1.99 \times 10^6$ | 92.04% |
| Background gel | $1.72 \times 10^5$ | 7.96% |

# 0 007 214

Conclusions

The following conclusions were reached relative to the quality control tests employed:

1. $^{99m}$Tc-pertechnetate was reduced by stannous chloride to a more reactive oxidation state (+4+5).

2. The reduced pertechnetate labeled both the TAG-1 and TAG-2 preparations.

3. The 99mTc-TAG-2 was tested for its ability to retain its activity and was found to retain its ability to react immunologically.

The use of radio-labeled TAG *in vivo* to detect cancer cells

Wistar rats were injected intracerebrally with Cisgliome tumor cells which had had previous passages in rats and in tissue culture. The rats were observed for the first signs of growing tumor, such as weakness, tremor or unsteadiness. These symptoms first appear seven to 10 days from injection, and with fast growing tumors result in death within three to four days in many animals, and one week in all. As soon as symptoms appeared, the animals were injected with labeled TAG intravenously in the tail vein, then the animal anesthetized at varying times, the brain removed, the tumor dissected free of normal brain, and the radioactivity in each dissected specimen compared.

### Preliminary $^{99m}$Tc-TAG experiment

| Animal | Sacrifice (hr post injection) | Tumor wt, mg | Counts/gm/min | |
| --- | --- | --- | --- | --- |
| | | | Tumor | Normal brain |
| A | 1.25 | 1.9 | 149,100 | 13,400 |
| B | 5.30 | 6.0 | 16,200 | 6,600 |
| C | 7.21 | 23.0 | 53,000 | 5,800 |
| D | 24.10 | 29.0 | 66,700 | 7,500 |

Tumor and normal brain specimens were counted overnight in the gamma-well counter. All samples and standards were decay corrected for convenience to the mid-count of the first sample in the sequence.

Conclusion

The preferential localization of radioactivity in tumor as compared to normal tissue is demonstrated above.

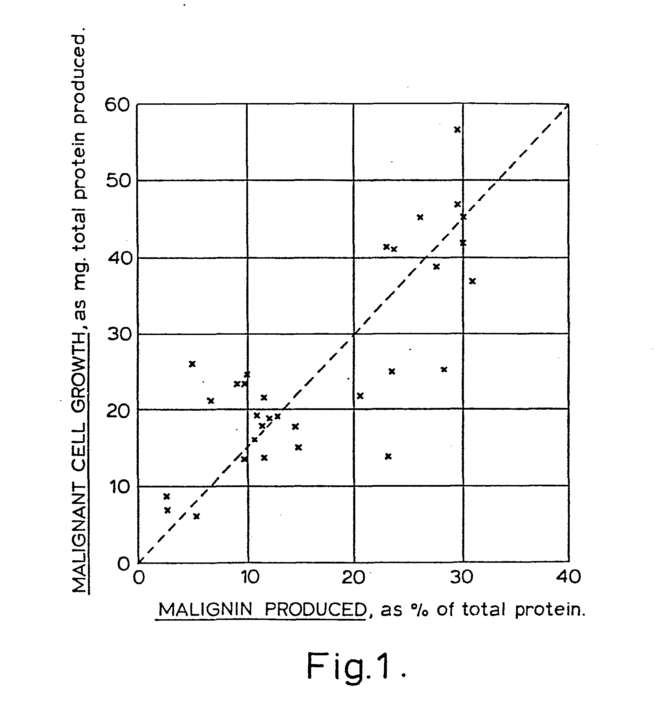

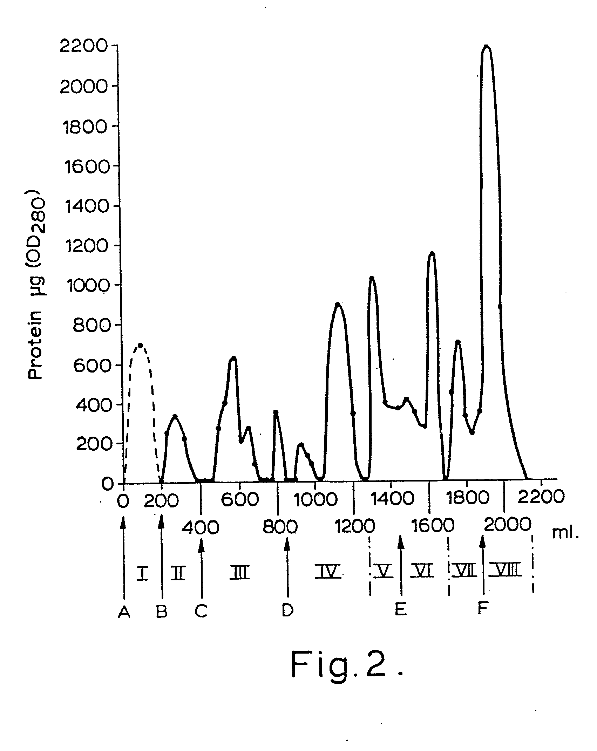

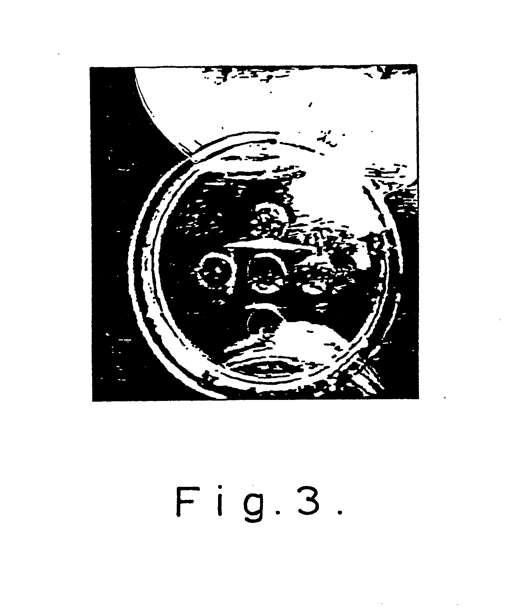

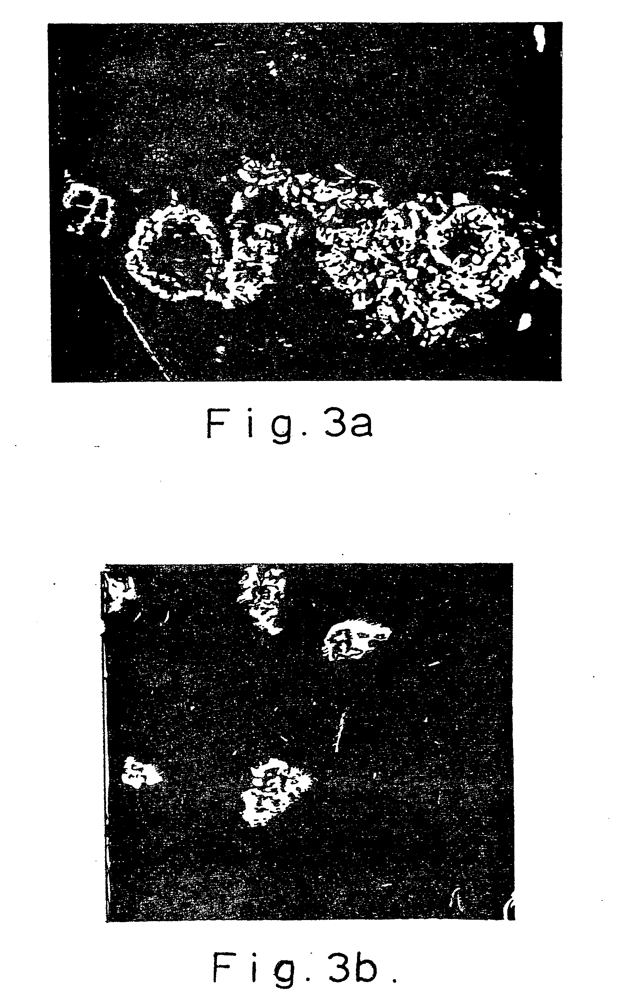

### Claims

1. A product for detecting the presence of cancerous or malignant tumor cells in a cell collection which comprises an anti-malignin TAG product (Target Attaching Globulins) in combination with a signal-emitting means which may be applied to said cell collection, whereby said anti-malignin TAG product preferentially attaches to cancerous or malignant cells, and detects said cancerous or malignant tumor cells by means of said signal-emitting means, characterised in that said signal-emitting means is $99^m$ Technetium.

2. A product as claimed in Claim 1, characterised in that said cancerous or malignant cells are glial tumor cells.

3. A product as claimed in Claim 1, characterised in that said cancerous or malignant cells are non-glial tumor cells.

4. A product as claimed in any of Claims 1 to 3, characterised in that said TAG product is produced by the reactiom of anti-malignin serum with a malignin-based reagent.

5. A product as claimed in Claim 4, characterised in that said malignin-based reagent is bromoacetylcellulose-malignin.

6. A product as claimed in Claim 1 characterised in that said Technetium product comprises $99^m$TC-pertechnate (sodium salt).

### Patentansprüche

1. Produkt für die Feststellung der Anwesenheit cancerogener oder maligner Tumorzellen in einer Zellkollektion, welches ein Antimalignin-TAG-Produkt (Target Attaching Globulins) in Kombination mit einem ein Signal emittierenden Mittel, welches an der Zellkollektion angebracht werden kann, umfaßt, wobei das Antimalignin-TAG-Produkt bevorzugt an cancerogene oder maligne Zellen gebunden ist, und die cancerogenen oder malignen Tumorzellen mittels des signalemittierenden Mittels nachweist, dadurch gekennzeichnet, daß das signalemittierende Mittel 99$^m$-Technetium ist.

8

2. Produkt nach Anspruch 1, dadurch gekennzeichnet, daß die cancerogenen oder malignen Zellen Glialtumorzellen sind.

3. Produkt nach Anspruch 1, dadurch gekennzeichnet, daß die cancerogenen oder malignen Zellen Nichtglialtumorzellen sind.

4. Produkt nach einem der Ansprüche 1 bis 3, dadurch gekennzeichnet, daß das TAG-Produkt durch Umsetzung von Antimaligninserum mit einem Reagens auf der Grundlage von Malignin hergestellt worden ist.

5. Produkt nach Anspruch 4, dadurch gekennzeichnet, daß das Reagens auf Maligningrundlage Bromacetylcellulosemalignin ist.

6. Produkt nach Anspruch 1, dadurch gekennzeichnet, daß das Technetiumprodukt 99$^m$TC-Pertechnat (Natriumsalz) enthält.

**Revendications**

1. Un produit pour la détection de la présence de cellules de tumeurs cancéreuses ou malignes dans un ensemble de cellules qui comprend un produit TAG (Targent Attaching Globulins) anti-malignine en combinaison avec un agent émettant un signal qui peut être appliqué audit ensemble de cellules, de façon à ce que leit produit TAG anti-malignine se fixe de préférence aux cellules cancéreuses ou malignes, et détecte lesdites cellules cancéreuses ou malignes au moyen agent émettant un signal, caractérisé en ce que ledit agent émettant un signal est le 99$^m$-technétium.

2. Un produit comme revendiqué dans la revendication 1 caractérisé en ce que lesdites cellules cancéreuses ou malignes sont des cellules de tumeur gliale.

3. Un produit comme revendiqué dans la revendication 1 caractérisé en ce que lesdites cellules cancéreuses ou malignes sont des cellules de tumeur non gliale.

4. Un produit comme revendiqué dans l'une quelconque des revendications 1 à 3, caractérisé en ce que ledit produit TAG est produit par réaction d'un sérum anti-malignine avec un réactif à base de malignine.

5. Un produit comme revendiqué dans la revendication 4 caractérisé en ce que ledit réactif à base de malignine est la bromoacétylcellulose-malignine.

6. Un produit comme revendiqué dans la revendication 1 caractérisé en ce que ledit produit à base de technétium comprend du 99$^m$Tc-pertechnétate (sel de sodium).

Fig.1.

Fig.2.

Fig. 3.

Fig. 3a

Fig. 3b.